# EUROPEAN PATENT APPLICATION

(11) **EP 4 119 557 A1**
(43) Date of publication of application: **18.01.2023**
(21) Application number: 21767461.3
(22) Date of filing: 10.03.2021
(51) Int. Cl.: C07D 471/04, A61K 31/519, A61P 35/00

(54) **PHARMACEUTICAL COMBINATION COMPRISING PYRIDINO[1,2-A]PYRIMIDINONE COMPOUND**

(30) Priority: 10.03.2020 CN 202010161911
(71) Applicant: CHIA TAI TIANQING PHARMACEUTICAL GROUP CO., LTD., Lianyungang, Jiangsu 222062 (CN)
(72) Inventor: YANG, Anqi, Nanjing, Jiangsu 211100 (CN); ZHANG, Xiquan, Nanjing, Jiangsu 211100 (CN); WANG, Xunqiang, Nanjing, Jiangsu 211100 (CN); YU, Ding, Nanjing, Jiangsu 211100 (CN); GUO, Fangfang, Nanjing, Jiangsu 211100 (CN)
(74) Representative: v. Bezold & Partner Patentanwälte - PartG mbB
(86) International application number: PCT/CN2021/079908
(87) International publication number: WO 2021/180111

(57) **Abstract**

The present invention belongs to the field of medicinal chemistry, and relates to a pharmaceutical combination comprising a pyridino[1,2-a]pyrimidinone compound. In particular, the pharmaceutical combination comprises a compound of formula **I** or a pharmaceutically acceptable salt thereof and a fulvestrant or a pharmaceutically acceptable salt thereof. The present invention also relates to the independent use of the pharmaceutical combination, or the compound of formula **I** or a pharmaceutically acceptable salt thereof in the treatment of breast cancer patients.

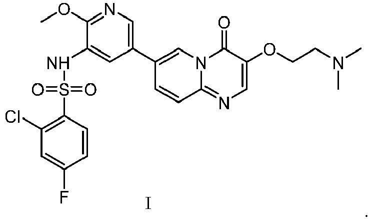

## Description

### CROSS-REFERENCE TO RELATED APPLICATIONS

The present application claims priority and benefit to the Chinese Patent Application No. 202010161911.8 filed with China National Intellectual Property Administration on Mar. 10, 2020, the disclosure of which is incorporated herein by reference in its entirety.

### TECHNICAL FIELD

The present application belongs to the field of medicinal chemistry, and relates to a pharmaceutical combination comprising a pyridino[1,2-a]pyrimidinone compound.

### BACKGROUND

PI3K pathway is a key signaling pathway where mutations are most commonly seen in cancer cells of human. It may lead to abnormal proliferation, activation and signal amplification of cells.

PI3K kinase (phosphatidylinositol-3-kinase, PI3Ks) belongs to the lipid kinase family and can phosphorylate the 3'-OH end of the inositol ring of phosphatidylinositol. The PI3K kinase is a lipid kinase consisting of a regulatory subunit p85 or p101 and a catalytic subunit p110, and activates downstream Akt and the like by catalyzing phosphorylation of phosphatidylinositol 4,5-bisphosphate (PIP2) to phosphatidylinositol 3,4,5-trisphosphate (PIP3), thereby playing a key role in proliferation, survival, metabolism and the like of cells. Therefore, inhibiting the phosphatidylinositol-3-kinase may affect the PI3K pathway, thereby inhibiting the proliferation and activation of cancer cells.

The tumor suppressor gene PTEN (phosphatase and tension homolog deleted on chromosome ten) enables PIP3 to be dephosphorylated to generate PIP2, thereby realizing negative regulation of PI3K/Akt signaling pathway, inhibiting proliferation of cells and promoting apoptosis of the cells. The frequent occurrences of PI3K gene mutation and amplification in cancers, absence of PTEN in cancers and the like all suggest that PI3K is closely related to tumorigenesis. The gene encoding PI3Kα is PIK3CA, and somatic missense mutations of PIK3CA increase the activity of PIK3α. Such mutations are found in tumor tissues, and are directly associated with malignant transformation of various tumors.

The incidence of breast cancer has increased rapidly in many countries over the past few decades (Minami 2004, Sim 2006, Yoo 2006, Matsuno 2007). Although a number of therapies have been developed, there has been no significant advance in existing therapies for many types of breast cancer. Thus, despite the numerous treatment options available for cancer patients, there is an urgent need for effective and safe therapeutic agents to treat cancer, and combination therapies applying multiple drugs are contemplated.

### SUMMARY

The present application provides a pharmaceutical combination comprising a compound of formula **I** or a pharmaceutically acceptable salt thereof, and fulvestrant or a pharmaceutically acceptable salt thereof,

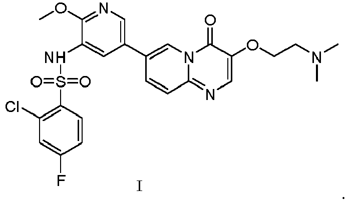

The present application provides a pharmaceutical combination for use in treating breast cancer, comprising a compound of formula **I** or a pharmaceutically acceptable salt thereof, and fulvestrant or a pharmaceutically acceptable salt thereof.

The present application provides a kit for use in treating breast cancer, comprising the pharmaceutical combination disclosed herein, and an instruction for use of the compound of formula **I** or the pharmaceutically acceptable salt thereof in combination with fulvestrant or the pharmaceutically acceptable salt thereof in treating breast cancer.

The present application provides use of the pharmaceutical combination described above in preparing a medicament for treating breast cancer in a patient.

The present application provides use of the pharmaceutical combination described above in treating breast cancer in a patient.

The present application provides a method for treating breast cancer in a patient, which comprises administering to the patient an effective amount of the pharmaceutical combination disclosed herein.

In another aspect, the present application provides a compound of formula **I** or a pharmaceutically acceptable salt thereof for use in treating breast cancer in a patient.

In another aspect, the present application provides a medicament comprising a compound of formula **I** or a pharmaceutically acceptable salt thereof as an active ingredient for use in treating breast cancer.

In another aspect, the present application provides use of a compound of formula **I** or a pharmaceutically acceptable salt thereof in preparing a medicament for treating breast cancer in a patient.

In another aspect, the present application provides use of the compound of formula **I** or the pharmaceutically acceptable salt thereof in treating breast cancer in a patient.

In another aspect, the present application provides a pharmaceutical composition for use in treating breast cancer, comprising a compound of formula **I** or a pharmaceutically acceptable salt thereof.

In another aspect, the present application provides a method for treating breast cancer in a patient, which comprises administering to the patient an effective amount of a compound of formula **I** or a pharmaceutically acceptable salt thereof, or a pharmaceutical composition thereof.

The compound of formula **I** described herein is shown below: 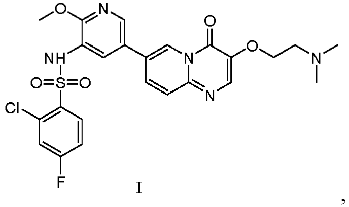 which is disclosed in WO2015192760.

### DETAILED DESCRIPTION OF INVENTION

The present application provides a pharmaceutical combination, comprising a compound of formula **I** or a pharmaceutically acceptable salt thereof, and fulvestrant or a pharmaceutically acceptable salt thereof.

In some embodiments of the present application, the pharmaceutical combination is for use in treating breast cancer in a patient. In some embodiments of the present application, the pharmaceutical combination is administered simultaneously, separately or sequentially for treating breast cancer in a patient. In some embodiments of the present application, a treatment cycle of the pharmaceutical combination is 2-6 weeks. In some embodiments of the present application, the pharmaceutical combination may be administered for 1, 2, 3, 4 or more treatment cycles.

The compound of formula **I** or the pharmaceutically acceptable salt thereof and fulvestrant or the pharmaceutically acceptable salt thereof may be identical or different in terms of administration regimen, dosage, route of administration and the like.

In some embodiments of the present application, in the pharmaceutical combination, the compound of formula **I** or the pharmaceutically acceptable salt thereof, or fulvestrant or the pharmaceutically acceptable salt thereof may be administered daily, for example, administered once, twice, three times, four times or more times daily; administered once every two, three, four, or five days; administered once every week, or every two, three, or four weeks; or administered once every month, or every two or more months.

In some embodiments of the present application, in the pharmaceutical combination, the compound of formula **I** or the pharmaceutically acceptable salt thereof is administered once daily to 3 times daily, or once every two days; fulvestrant or the pharmaceutically acceptable salt thereof is administered once on days 1 and 15 (if available) in the first treatment cycle, and once on day 1 in each subsequent treatment cycle (if there are subsequent treatment cycles).

In some embodiments of the present application, in the pharmaceutical combination, the compound of formula **I** or the pharmaceutically acceptable salt thereof is administered at a dose selected from 1-100 mg, preferably 5-50 mg, more preferably 10-50 mg, even more preferably 10-40 mg, still more preferably 10-15 mg, and most preferably 20-30 mg; fulvestrant or the pharmaceutically acceptable salt thereof is administered at a dose selected from 250-1000 mg, and preferably 250-500 mg.

In some embodiments of the present application, in the pharmaceutical combination, the compound of formula **I** or the pharmaceutically acceptable salt thereof is administered at a daily dose selected from 1-100 mg, preferably 5-50 mg, more preferably 10-50 mg, even more preferably 10-40 mg, and most preferably 20-30 mg; fulvestrant or the pharmaceutically acceptable salt thereof is administered at a dose selected from 250-1000 mg, and preferably 250-500 mg.

In some embodiments of the present application, within a single treatment cycle, the pharmaceutical combination comprises the compound of formula **I** or the pharmaceutically acceptable salt thereof and fulvestrant or the pharmaceutically acceptable salt thereof in a mass ratio of (0.01-6):1, preferably (0.05-3):1, more preferably (0.1-3):1, even more preferably (0.2-2.5):1, still more preferably (0.3-2.0):1, and most preferably (0.5-1.8):1.

In some embodiments of the present application, within a single treatment cycle, the pharmaceutical combination comprises the compound of formula **I** or the pharmaceutically acceptable salt thereof and fulvestrant or the pharmaceutically acceptable salt thereof in a mass ratio of (0.5-1.2):1, and preferably (0.56-1.12):1.

In some embodiments of the present application, in the pharmaceutical combination, the compound of formula **I** or the pharmaceutically acceptable salt thereof and fulvestrant or the pharmaceutically acceptable salt thereof are each in a form of a pharmaceutical composition.

In some embodiments of the present application, in the pharmaceutical combination, the compound of formula **I** or the pharmaceutically acceptable salt thereof and fulvestrant or the pharmaceutically acceptable salt thereof are each in a form of a pharmaceutical composition and can be administered simultaneously, sequentially, or at intervals.

In some embodiments of the present application, the pharmaceutical combination comprises a pharmaceutical composition of the compound of formula **I** or the pharmaceutically acceptable salt thereof at a single dose of 1-50 mg, and a pharmaceutical composition of fulvestrant or the pharmaceutically acceptable salt thereof at a single dose of 250-500 mg; preferably, the pharmaceutical composition of the compound of formula **I** or the pharmaceutically acceptable salt thereof has a single dose of 1 mg, 5 mg, or 20 mg.

In some embodiments of the present application, in the pharmaceutical combination, the compound of formula **I** or the pharmaceutically acceptable salt thereof and fulvestrant or the pharmaceutically acceptable salt thereof are each administered in a single dose or multiple doses.

In some embodiments of the present application, the pharmaceutical combination is packaged in a kit, wherein the kit further comprises an instruction for use of the compound of formula **I** or the pharmaceutically acceptable salt thereof in combination with fulvestrant or the pharmaceutically acceptable salt thereof for treating breast cancer in a patient.

In some embodiments of the present application, in the pharmaceutical combination, the compound of formula **I** or the pharmaceutically acceptable salt thereof and fulvestrant or the pharmaceutically acceptable salt thereof are separately packaged in their kits, wherein the kits further comprise an instruction for use of the compound of formula **I** or the pharmaceutically acceptable salt thereof in combination with fulvestrant or the pharmaceutically acceptable salt thereof for treating breast cancer in a patient.

The present application provides a kit, comprising the pharmaceutical combination disclosed herein, and an instruction for use of the compound of formula **I** or the pharmaceutically acceptable salt thereof in combination with fulvestrant or the pharmaceutically acceptable salt thereof in treating breast cancer.

The present application provides a kit for use in treating breast cancer, comprising the pharmaceutical combination disclosed herein, and an instruction for use of the compound of formula **I** or the pharmaceutically acceptable salt thereof in combination with fulvestrant or the pharmaceutically acceptable salt thereof in treating breast cancer.

The present application provides use of the pharmaceutical combination described above in preparing a medicament for treating breast cancer in a patient.

The present application provides use of the pharmaceutical combination described above in treating breast cancer in a patient.

The present application provides a method for treating breast cancer in a patient, which comprises administering to the patient an effective amount of the pharmaceutical combination disclosed herein.

In another aspect, the present application provides a compound of formula **I** or a pharmaceutically acceptable salt thereof for use in treating breast cancer in a patient.

In another aspect, the present application provides a medicament comprising a compound of formula **I** or a pharmaceutically acceptable salt thereof as an active ingredient for use in treating breast cancer.

In another aspect, the present application provides use of a compound of formula **I** or a pharmaceutically acceptable salt thereof in preparing a medicament for treating breast cancer in a patient.

In another aspect, the present application provides use of the compound of formula **I** or the pharmaceutically acceptable salt thereof in treating breast cancer in a patient.

In another aspect, the present application provides a pharmaceutical composition for use in treating breast cancer, comprising a compound of formula **I** or a pharmaceutically acceptable salt thereof.

In another aspect, the present application provides a method for treating breast cancer in a patient, which comprises administering to the patient an effective amount of a compound of formula **I** or a pharmaceutically acceptable salt thereof, or a pharmaceutical composition thereof.

### Compound of formula I or pharmaceutically acceptable salt thereof, or pharmaceutical composition thereof

The compound of formula **I** described herein is shown below: 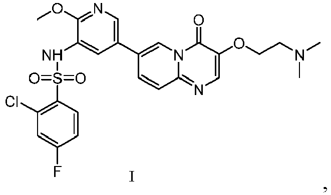 which is disclosed in WO2015192760.

The pharmaceutical composition of the compound of formula **I** or the pharmaceutically acceptable salt thereof may be in any suitable dosage form including, but not limited to, tablet, lozenge, pill, capsule (e.g., hard capsule, soft capsule, enteric capsule and microcapsule), elixir, granule, syrup, injection (intramuscular, intravenous and intraperitoneal), granule, emulsion, suspension, solution, dispersion and dosage forms of sustained-release formulations for oral or non-oral administration.

The pharmaceutical composition of the compound of formula **I** or the pharmaceutically acceptable salt thereof may comprise a pharmaceutically acceptable carrier and/or excipient.

In some embodiments of the present application, a single dose of the pharmaceutical composition of the compound of formula **I** or the pharmaceutically acceptable salt thereof comprises 1-50 mg of the compound of formula **I** or the pharmaceutically acceptable salt thereof (on the basis of the compound of formula **I**); preferably, the single dose comprises 1 mg, 5 mg or 20 mg.

In some embodiments of the present application, the pharmaceutical composition of the compound of formula **I** or the pharmaceutically acceptable salt thereof is preferably selected from formulations suitable for oral administration, such as tablets. For example, the pharmaceutical composition of the compound of formula **I** or the pharmaceutically acceptable salt thereof can be a tablet comprising the compound of formula **I** or the pharmaceutically acceptable salt thereof and a pharmaceutically acceptable carrier and/or excipient.

In some embodiments of the present application, the pharmaceutical composition of the compound of formula **I** or the pharmaceutically acceptable salt thereof may be a tablet comprising the compound of formula **I** or the pharmaceutically acceptable salt thereof, microcrystalline cellulose, mannitol, croscarmellose sodium, hydroxypropyl methylcellulose and magnesium stearate.

### Fulvestrant or pharmaceutically acceptable salt thereof, or pharmaceutical composition thereof

Fulvestrant is disclosed in U.S. Patent No. 4,659,516 and has the following chemical structure:

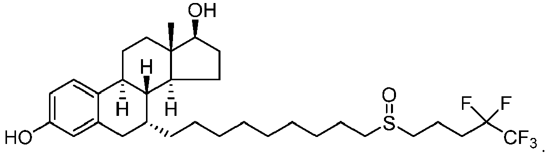

The pharmaceutical composition of fulvestrant or the pharmaceutically acceptable salt thereof may be in any suitable dosage form including, but not limited to, tablet, lozenge, pill, capsule (e.g., hard capsule, soft capsule, enteric capsule and microcapsule), elixir, granule, syrup, injection (intramuscular, intravenous and intraperitoneal), granule, emulsion, suspension, solution, dispersion and dosage forms of sustained-release formulations for oral or non-oral administration.

The pharmaceutical composition of fulvestrant or the pharmaceutically acceptable salt thereof may comprise a pharmaceutically acceptable carrier and/or excipient.

Fulvestrant may be administered in any commercially available form.

In some embodiments of the present application, the pharmaceutical composition of fulvestrant or the pharmaceutically acceptable salt thereof is an injection at a single dose of 250-1000 mg, preferably 250-500 mg (calculated on the basis of fulvestrant).

### Breast cancer

In some embodiments of the present application, the breast cancer is hormone receptor (HR)-positive. In some embodiments of the present application, the HR-positive includes estrogen receptor (ER)-positive or progesterone receptor (PR)-positive.

In some embodiments of the present application, the breast cancer is human epidermal growth factor receptor 2 (HER2)-negative.

In some embodiments of the present application, the breast cancer has a PIK3CA gene alteration. In some embodiments of the present application, the PIK3CA gene alteration includes a PIK3CA gene mutation or a PIK3CA gene amplification.

In some embodiments of the present application, the PIK3CA gene alteration occurs in any exon region of the PIK3CA gene. In some embodiments of the present application, the PIK3CA gene alteration includes those occurring in region of exon 1, 2, 5, 7, 9 or 20 in the PIK3CA gene. In some embodiments of the present application, the PIK3CA gene alteration includes one or more of the following amino acid mutations: K111N, R263Q, R277W, R278W, K331E, K333N, G353D, E1093K, C1258R, E1624K, E1633K, E1634G, Q1636K, H3140K, H3140R, H3140L, H3139Y, H1047R, C420R, H1047L, E542K, E545K or Q546R, R88Q, N345K, E545Q, H1047Q and I143V.

In some embodiments of the present application, the breast cancer is a locally advanced recurrent and/or metastatic breast cancer.

In some embodiments of the present application, the breast cancer is an unresectable breast cancer.

In some embodiments of the present application, the patient with breast cancer is a postmenopausal female patient with breast cancer.

In some embodiments of the present application, the patient with breast cancer is a HR-positive, HER2-negative, PIK3CA gene altered, postmenopausal female patient with locally advanced recurrent and/or metastatic breast cancer.

In some embodiments of the present application, the patient with breast cancer has previously received treatment with one or two or more prior therapeutic regimens. In some embodiments of the present application, the patient with breast cancer has previously received treatment with one, two, three, four or five prior therapeutic regimens. In some embodiments of the present application, the prior therapeutic regimen for breast cancer includes pharmacotherapy and radiotherapy. In some embodiments of the present application, the pharmacotherapy includes chemotherapy, and neoadjuvant or adjuvant endocrine therapy.

In some embodiments of the present application, the neoadjuvant or adjuvant endocrine therapy includes use of an antiestrogen or an aromatase inhibitor (AI).

In some embodiments of the present application, the antiestrogen is selected from the group consisting of tamoxifen, toremifene, raloxifene, acolbifene and endoxifen.

In some embodiments of the present application, the aromatase inhibitor is selected from the group consisting of anastrozole, letrozole, exemestane and formestane.

In some embodiments of the present application, the drug used in the pharmacotherapy of prior therapeutic regimen for breast cancer is selected from one or more of the group consisting of: adriamycin, epirubicin, pirarubicin, cyclophosphamide, ifosfamide, methotrexate, paclitaxel, albumin-bound paclitaxel, docetaxel, vinorelbine, vincristine, gemcitabine, capecitabine, etoposide, eribulin, utidelone, cisplatin, carboplatin, fluorouracil, bevacizumab, trastuzumab, pertuzumab, goserelin, tamoxifen, raloxifene, acolbifene, endoxifen, anastrozole, letrozole, exemestane and formestane.

In some embodiments of the present application, the chemotherapy regimens of the prior therapy for breast cancer includes, but is not limited to: AC regimen, AC-T regimen, AT regimen, AT-NP regimen, EC regimen, FAC regimen, FEC-T regimen, GP regimen, GT regimen, NP regimen, NX regimen, TAC regimen, T + bevacizumab regimen, TC regimen, TP regimen, TX regimen and X + bevacizumab regimen.

In some embodiments of the present application, the patient with breast cancer has previously received neoadjuvant or adjuvant endocrine therapy.

In some embodiments of the present application, the patient with breast cancer has previously received neoadjuvant or adjuvant endocrine therapy, had radiologically confirmed disease progression (according to RECIST 1.1 criteria) during treatment or disease recurrence within 12 months after the end of treatment and has not received further endocrine therapy after the disease progression or disease recurrence.

In some embodiments of the present application, the patient with breast cancer experienced recurrence after prior treatment with the antiestrogen or the aromatase inhibitor as the first-line endocrine therapy for advanced disease with radiologically confirmed progression (according to RECIST 1.1 criteria).

In some embodiments of the present application, the patient with breast cancer experienced recurrence more than 12 months after receiving the neoadjuvant or adjuvant endocrine therapy, and then experienced recurrence again after receiving the antiestrogen or the aromatase inhibitor as the first-line endocrine therapy for advanced disease with radiologically confirmed progression (according to RECIST 1.1 criteria).

In some embodiments of the present application, the breast cancer is a primary metastatic disease, and the patient with breast cancer experienced recurrence after previously receiving the antiestrogen or the aromatase inhibitor as the first-line endocrine therapy for metastatic disease with radiologically confirmed progression (according to RECIST 1.1 criteria).

In some embodiments of the present application, the breast cancer optionally includes a combination of any two or more of the above types of breast cancer.

### Treatment regimen

In some embodiments of the present application, a treatment cycle for treating breast cancer in a patient with the pharmaceutical combination disclosed herein is 2-6 weeks. In some embodiments of the present application, the treatment cycle for treating breast cancer in a patient is 2 weeks, 3 weeks, 4 weeks, 5 weeks, 6 weeks, or a range formed by any of the above values. In some embodiments of the present application, the treatment cycle for treating breast cancer in a patient is 4 weeks.

In some embodiments of the present application, the pharmaceutical combination may be administered for 1, 2, 3, 4 or more treatment cycles.

In some embodiments of the present application, in the pharmaceutical combination, the compound of formula **I** or the pharmaceutically acceptable salt thereof, or fulvestrant or the pharmaceutically acceptable salt thereof may be administered daily, for example, administered once, twice, three times, four times or more times daily; administered once every two, three, four, or five days; administered once every week, or every two, three, or four weeks; or administered once every month, or every two or more months.

In some embodiments of the present application, in the treatment of breast cancer in a patient, the compound of formula **I** or the pharmaceutically acceptable salt thereof is administered once daily to 3 times daily, or once every two days; preferably once daily.

In some embodiments of the present application, in the treatment of breast cancer in a patient, fulvestrant or the pharmaceutically acceptable salt thereof is administered once on days 1 and 15 (if available) in the first treatment cycle, and once on day 1 in each subsequent treatment cycle (if there are subsequent treatment cycles).

For example, when the treatment cycle is 4 weeks, in the treatment of breast cancer in a patient, fulvestrant or the pharmaceutically acceptable salt thereof is administered once on days 1 and 15 in the first treatment cycle, and once on day 1 in each subsequent treatment cycle if there are subsequent treatment cycles.

In some embodiments of the present application, in the treatment of breast cancer in a patient, the compound of formula **I** or the pharmaceutically acceptable salt thereof is administered to the patient at a daily dose selected from 1-100 mg. In some embodiments of the present application, in the treatment of breast cancer in a patient, the compound of formula **I** or the pharmaceutically acceptable salt thereof is administered to the patient at a daily dose selected from the group consisting of 1 mg, 2 mg, 3 mg, 4 mg, 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, 20 mg, 21 mg, 22 mg, 23 mg, 24 mg, 25 mg, 26 mg, 27 mg, 28 mg, 29 mg, 30 mg, 31 mg, 32 mg, 33 mg, 34 mg, 35 mg, 36 mg, 37 mg, 38 mg, 39 mg, 40 mg, 41 mg, 42 mg, 43 mg, 44 mg, 45 mg, 46 mg, 47 mg, 48 mg, 49 mg, 50 mg, 51 mg, 52 mg, 53 mg, 54 mg, 55 mg, 56 mg, 57 mg, 58 mg, 59 mg, 60 mg, 61 mg, 62 mg, 63 mg, 64 mg, 65 mg, 66 mg, 67 mg, 68 mg, 69 mg, 70 mg, 71 mg, 72 mg, 73 mg, 74 mg, 75 mg, 76 mg, 77 mg, 78 mg, 79 mg, 80 mg, 81 mg, 82 mg, 83 mg, 84 mg, 85 mg, 86 mg, 87 mg, 88 mg, 89 mg, 90 mg, 91 mg, 92 mg, 93 mg, 94 mg, 95 mg, 96 mg, 97 mg, 98 mg, 99 mg, 100 mg and a range formed by any of the above values. In some embodiments of the present application, in the treatment of breast cancer in a patient, the compound of formula **I** or the pharmaceutically acceptable salt thereof is administered to the patient at a daily dose selected from the group consisting of 5-50 mg, 10-50 mg, 10-40 mg and 20-30 mg.

In some embodiments of the present application, in the treatment of breast cancer in a patient, the compound of formula **I** or the pharmaceutically acceptable salt thereof is administered to the patient at a dose selected from 1-100 mg. In some embodiments of the present application, in the treatment of breast cancer in a patient, the compound of formula **I** or the pharmaceutically acceptable salt thereof is administered to the patient at a dose selected from the group consisting of 1 mg, 2 mg, 3 mg, 4 mg, 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, 20 mg, 21 mg, 22 mg, 23 mg, 24 mg, 25 mg, 26 mg, 27 mg, 28 mg, 29 mg, 30 mg, 31 mg, 32 mg, 33 mg, 34 mg, 35 mg, 36 mg, 37 mg, 38 mg, 39 mg, 40 mg, 41 mg, 42 mg, 43 mg, 44 mg, 45 mg, 46 mg, 47 mg, 48 mg, 49 mg, 50 mg, 51 mg, 52 mg, 53 mg, 54 mg, 55 mg, 56 mg, 57 mg, 58 mg, 59 mg, 60 mg, 61 mg, 62 mg, 63 mg, 64 mg, 65 mg, 66 mg, 67 mg, 68 mg, 69 mg, 70 mg, 71 mg, 72 mg, 73 mg, 74 mg, 75 mg, 76 mg, 77 mg, 78 mg, 79 mg, 80 mg, 81 mg, 82 mg, 83 mg, 84 mg, 85 mg, 86 mg, 87 mg, 88 mg, 89 mg, 90 mg, 91 mg, 92 mg, 93 mg, 94 mg, 95 mg, 96 mg, 97 mg, 98 mg, 99 mg, 100 mg and a range formed by any of the above values. In some embodiments of the present application, in the treatment of breast cancer in a patient, the compound of formula **I** or the pharmaceutically acceptable salt thereof is administered to the patient at a dose selected from the group consisting of 5-50 mg, 10-50 mg, 10-40 mg, 10-15 mg and 20-30 mg.

In some embodiments of the present application, in the treatment of breast cancer in a patient, fulvestrant or the pharmaceutically acceptable salt thereof is administered to the patient at a dose selected from 250-1000 mg, preferably 250-500 mg.

In some embodiments of the present application, the compound of formula **I** or the pharmaceutically acceptable salt thereof is administered simultaneously with fulvestrant or the pharmaceutically acceptable salt thereof, or fulvestrant or the pharmaceutically acceptable salt thereof is administered within 0.5 h after administration of the compound of formula **I** or the pharmaceutically acceptable salt thereof.

In some embodiments of the present application, the compound of formula **I** or the pharmaceutically acceptable salt thereof is administered orally at fasting, and fulvestrant or the pharmaceutically acceptable salt thereof is administered by intramuscular injection.

As used herein, the foregoing treatment regimen is applicable to the compound of formula **I** or the pharmaceutically acceptable salt thereof, fulvestrant or the pharmaceutically acceptable salt thereof, the pharmaceutical combination, the kit, or the use in or method for treating breast cancer in a patient as described herein.

### Administration

The content below is not intended to limit the administration of the pharmaceutical combination disclosed herein. The components in the pharmaceutical combination disclosed herein can be administered independently, or some or all of the components are co-administered in various proper routes, including, but not limited to, oral administration or parenteral administration (by intravenous, intramuscular, local or subcutaneous routes). In some embodiments, the components in the pharmaceutical combination disclosed herein can be administered independently, or some or all of the components are co-administered by means of oral administration or injection, for example, intravenous injection or intraperitoneal injection.

The components of the pharmaceutical combination of the present application can be independent suitable dosage forms, or some or all of the components are co-formulated in a suitable dosage form including but not limited to, tablet, lozenge, pill, capsule (for example, hard capsule, soft capsule, enteric capsule and microcapsule), elixir, granule, syrup, injection (intramuscular, intravenous and intraperitoneal), granule, emulsion, suspension, solution, dispersion and dosage forms of sustained-release formulations for oral or non-oral administration.

The components in the pharmaceutical combination disclosed herein can be formulated independently, or some or all of the components are co-formulated with a pharmaceutically acceptable carrier and/or excipient.

### Technical effects

The pharmaceutical combination disclosed herein has good anti-tumor activity, especially anti-breast cancer activity. The pharmaceutical combination disclosed herein has good safety.

In addition, the present application demonstrates that the compound of formula **I** or the pharmaceutically acceptable salt thereof is effective in treating breast cancer. The compound of formula **I** or the pharmaceutically acceptable salt thereof described herein has good safety.

Furthermore, the compound of formula **I** or the pharmaceutically acceptable salt thereof, or the pharmaceutical combination of the present application will help provide:
(1) favorable efficacy in reducing the growth of tumors or even eliminating the tumors;
(2) treatment that is well tolerated in a patient with fewer adverse effects and/or complications;
(3) good disease control rate in treated patients;
(4) prolonged survival (e.g., median survival, progression-free survival, or overall survival) in treated patient;
(5) prolonged survival (e.g., median survival, progression-free survival, or overall survival) in treated patient as compared with those receiving standard chemotherapy;
(6) longer duration of response (DOR); and/or
(7) favorable activity in treating breast cancer and better anti-tumor synergistic effect, as compared with either monotherapy of the drug of the combination.

### Definitions and description

Unless otherwise stated, the following terms used herein shall have the following meanings. A certain term, unless otherwise specifically defined, should not be considered uncertain or unclear, but construed according to its common meaning in the field. When referring to a trade name, it is intended to refer to its corresponding commercial product or its active ingredient.

As used herein, unless otherwise stated, the terms "comprise", "comprises" and "comprising" or equivalents thereof are open-ended statements and mean that elements, components and steps that are not specified may be included in addition to those listed.

All patents, patent applications and other identified publications are explicitly incorporated herein by reference for the purpose of description and disclosure. Any reference to these publications herein shall not be construed as an admission that the publications form part of the commonly recognized knowledge in the art.

The term "combination" refers to a fixed combination, or a non-fixed combination (or a kit) in one dosage unit form for combined administration, wherein the compound of formula **I** and the other active ingredient in the combination can be administered simultaneously alone, or separately at certain time intervals. The term "fixed combination" refers to simultaneous administration of the active ingredients such as the compound of formula **I** and the other active ingredient in the combination to a patient in the form of a single substance or dose. The term "non-fixed combination" or "kit" refers to simultaneous or sequential (without specific time limitation) administrations of the active ingredients such as the compound of formula **I** and the other active ingredient in the combination to a patient as independent substances.

The terms "co-administration" or "combined administration" and the like are intended to encompass administrations of selected therapeutic agents (i.e., active ingredients) to a single patient, and are intended to encompass therapies in which the therapeutic agents are not necessarily administered by the same route of administration or administered simultaneously.

The term "pharmaceutically acceptable" is used herein for those compounds, materials, compositions, and/or dosage forms which are, within the scope of sound medical judgment, suitable for use in contact with the tissues of human beings and animals without excessive toxicity, irritation, allergic response, or other problems or complications, and commensurate with a reasonable benefit/risk ratio.

The term "pharmaceutically acceptable salt" includes salts formed from basic radicals and free acids and salts formed from acidic radicals and free bases.

As used herein, the content of the compound of formula **I** or the pharmaceutically acceptable salt thereof and fulvestrant or the pharmaceutically acceptable salt thereof, e.g., the amount administered, the dose, the amount in the pharmaceutical composition, are calculated based on the free base form.

As used herein, if the compound in the pharmaceutical combination has, for example, at least one basic site, an acid addition salt may be formed. If needed, an acid addition salt corresponding to additional basic sites may also be formed. A compound with at least one acidic group (for example, -COOH) can further form a salt with a base. A compound, for example, comprising both carboxyl and amino, can further form an inner salt.

The term "patient" is a mammal, such as a human, dog, cow, horse, pig, sheep, goat, cat, mouse, rabbit, rat or transgenic non-human animal. In some embodiments, the patient is a human.

The term "pharmaceutical composition" refers to a mixture consisting of one or more of the compounds or the pharmaceutically acceptable salts thereof described herein, or the pharmaceutical combinations thereof disclosed herein and a pharmaceutically acceptable excipient. The pharmaceutical composition is intended to facilitate the administration of the compound of the present application or the pharmaceutical combination thereof disclosed herein to a patient.

The term "treatment" generally refers to obtaining desired pharmacological and/or physiological effects, including partially or completely stabilizing or curing a disease and/or an effect that the disease has. As used herein, "treatment" encompasses any treatment of a disease in a patient, including (a) inhibiting a symptom of the disease, i.e., blocking the progression of the disease; or (b) alleviating a symptom of the disease, i.e., causing remission of the disease or the symptom.

The term "effective amount" refers to an amount of the compound of the present application for (i) treating a specific disease, condition or disorder, or (ii) alleviating, ameliorating or eliminating one or more symptoms of a specific disease, condition or disorder. The amount of the compound of the present application composing the "therapeutically effective amount" varies dependently on the compound, the disease state and its severity, the route of administration, and the age of the mammal to be treated, but can be determined routinely by those skilled in the art in accordance with their knowledge and the present disclosure.

The term "single dose" refers to the smallest unit of packaging containing a certain quantity of pharmaceutical product; for example, each tablet of drug is a single dose; in a box of seven capsules, each capsule is a single dose; or a vial of injection is a single dose.

In the context of cancer, the term "relapse" and "recurrent" means that a disease reoccurs after objective response is achieved due to treatment with a certain therapeutic regimen. For breast cancer, recurrence can be defined as meeting any of the following criteria: 1. the patient has previously received a first-line systemic endocrine therapy for advanced disease or metastatic disease, and had radiologically confirmed disease progression after the treatment; 2. the patient has previously received neoadjuvant or adjuvant endocrine therapy, and had radiologically confirmed disease progression within 12 months after the treatment. "Objective response" includes complete response and partial response.

In the context of cancer, the term "local recurrence" refers to recurrence in the same location as the primary lesion or near the primary lesion after the objective response is achieved.

In the context of cancer, the term "locally advanced" refers to the spread of the primary lesion to nearby tissues or lymph nodes.

As used herein, with respect to drugs used in the prior therapeutic regimen, reference may be made to the following, or treatment guidelines or textbooks relating to medicine and pharmacy:
AC regimen: anthracyclines in combination with cyclophosphamide, such as doxorubicin or epirubicin, and cyclophosphamide.
AC-T regimen: anthracyclines in combination with cyclophosphamide followed by taxanes, such as doxorubicin or epirubicin, cyclophosphamide, and paclitaxel or docetaxel.
AT regimen: anthracyclines in combination with taxanes, such as doxorubicin or epirubicin, and docetaxel.
AT-NP regimen: anthracyclines in combination with taxanes followed by platinum-based drugs, such as doxorubicin or epirubicin, docetaxel, vinorelbine, and cisplatin.
EC regimen: epirubicin, and cyclophosphamide.
FAC regimen: fluorouracil, doxorubicin, and cyclophosphamide.
FEC-T regimen: fluorouracil, epirubicin, cyclophosphamide, and docetaxel.
GP regimen: gemcitabine, and cisplatin or carboplatin.
GT regimen: gemcitabine, and paclitaxel.
NP regimen: vinorelbine, and cisplatin or carboplatin.
NX regimen: vinorelbine, and capecitabine.
TAC regimen: docetaxel, doxorubicin, and cyclophosphamide.
T + bevacizumab regimen: albumin-bound paclitaxel, and bevacizumab.
TC regimen: docetaxel, and cyclophosphamide.
TP regimen: taxanes in combination with platinum-based drugs, such as albumin-bound paclitaxel, and cisplatin or carboplatin.
TX regimen: taxanes in combination with capecitabine, such as docetaxel or albumin-bound paclitaxel, and capecitabine.
X + bevacizumab regimen: capecitabine, and bevacizumab.

As used herein, the chemotherapies belong to the prior art in this filed. Those skilled in the art would readily refer to treatment guidelines or related medical and pharmaceutical textbooks in the prior art (e.g., Chinese Society of Clinical Oncology (CSCO) diagnosis and treatment guidelines for breast cancer 2020) for details of a chemotherapy regimen (including but not limited to the drugs used, the dosage, or treatment cycle). The above examples of drugs used for the chemotherapies in the present application are exemplary, and reference shall be made to treatment guidelines or related medical and pharmaceutical textbooks for the details of chemotherapies (including but not limited to the drug used, the dosage, or treatment cycle).

All patents, patent applications and other identified publications are explicitly incorporated herein by reference for the purpose of description and disclosure. Any reference to these publications herein shall not be construed as an admission that the publications form part of the commonly recognized knowledge in the art.

### DETAILED DESCRIPTION

The present invention will be illustrated in more detail through specific examples. The following examples are provided for illustrative purposes only, and are not intended to limit the present invention in any way.

The compound of formula **I** described in the examples is as follows: 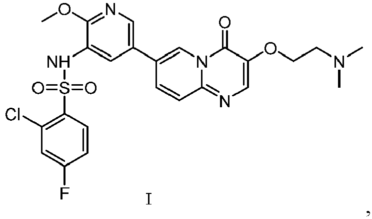 of which the preparation method is disclosed in WO2015192760.

### Example 1 Tablets of the compound of formula I

**Table 1 Formulation of tablets of the compound of formula I**

| Component | Amount | | | | | |
|---|---|---|---|---|---|---|
| | Specification 5 mg | | Specification 20 mg | | Specification 1 mg | |
| | mg | % | mg | % | mg | % |
| Compound of formula I | 5.0 | 5.0 | 20.0 | 5.0 | 1.0 | 1.0 |
| Microcrystalline cellulose | 25.0 | 25.0 | 100.0 | 25.0 | 25.0 | 25.0 |
| Mannitol | 63.0 | 63.0 | 252.0 | 63.0 | 67.0 | 67.0 |
| Croscarmellose sodium | 5 | 5 | 20 | 5 | 5 | 5 |
| Hydroxypropyl methylcellulose | 1.0 | 1.0 | 4.0 | 1.0 | 1.0 | 1.0 |
| Magnesium stearate | 1.0 | 1.0 | 4.0 | 1.0 | 1.0 | 1.0 |
| Weight of core tablet | 100 | 100 | 400 | 100 | 100 | 100 |

Preparation method:
1) The compound of formula **I,** microcrystalline cellulose, mannitol and croscarmellose sodium were each fed into a grinding and sizing machine successively and then sieved, and the materials were then collected and premixed to obtain a premixed material.
2) Hydroxypropyl methylcellulose was formulated into an aqueous solution to be used as a binder.
3) The premixed material in step 1) was transferred into a wet granulation pot, and the binder obtained in step 2) was added for granulation.
4) The soft and wet materials obtained were subjected to sizing and drying, then magnesium stearate was added, the materials were mixed.
5) Tableting was performed.

Optionally, the resulting tablets were coated.

The compound of formula **I** is prepared according to the method disclosed in WO2015192760.

### Example 2 Clinical trials for breast cancer

### 2.1 Test compound and administration regimen

### Tablet of the compound of formula I: 5 mg/tablet and 20 mg/tablet

The tablets were administered orally once daily at fasting for 4 weeks, i.e., in treatment cycles of 4 weeks (28 days). If there were no special circumstances, it was recommended to take it at a fixed time every day. During the treatment period, a missed dose should not be supplemented if the interval before the next dose was shorter than 12 hours.

### Fulvestrant injection: 250 mg/5 mL

The fulvestrant injection was administered by slow intragluteal injection (which must be given within 0.5 hours after administration of the tablet of compound of formula **I**) at a fixed dose of 500 mg on days 1 and 15 in first 4-week (28-day) treatment cycle, and on day 1 in each subsequent treatment cycle. Two vials of 5-mL injection were administered, one in each side of the buttock (1-2 min/5 mL).

### 2.2 Inclusion criteria

Subjects: HR-positive, HER2-negative, tumor PIK3CA gene altered (mutated or amplified), postmenopausal female subjects with locally advanced recurrent and/or metastatic breast cancer.

Subjects who met all of the following inclusion criteria could be enrolled in this trial:
1) Histopathologically confirmed breast cancer.
2) hormone receptor (HR) (estrogen receptor (ER) or progesterone receptor (PR))-positive and human epidermal growth factor receptor-2 (HER2)-negative of primary or metastatic tumors as confirmed by immunohistochemical analysis.
3) Agreement to provide 10 fresh unstained sections of tumor tissue (surgery or biopsy) and 10 mL of peripheral blood for gene mutation detection; the subjects must have a sample (tissue section or blood) positive for PIK3CA gene alteration (mutation or amplification) to be enrolled.
4) Aged ≥ 18 years; postmenopausal females with any of the following postmenopausal statuses:
   a) Aged ≥ 60 years.
   b) Aged < 60 years, with recorded levels of follicle-stimulating hormone and estradiol within the postmenopausal range, and for 12 months of menopause without receiving chemotherapy, tamoxifen, toremifene or ovarian suppression therapy.
   c) Previously received bilateral ovariectomy (at least 4 weeks from the first dose).
   d) In menopausal state by taking castration medicine (GnRHa) such as goserelin (Zoladex), leuprorelin (Enanton) to achieve ovarian suppression.
5) Patients with unresectable, locally advanced recurrent and/or metastatic tumors having at least one measurable lesion (long diameter of measurable lesion ≥ 10 mm or short diameter of enlarged lymph nodes ≥ 15 mm by spiral CT scan, according to RECIST version 1.1).
6) A disease that is not amenable to radical resection or radiation therapy; in addition, any of the following must be met:
   a) Previously received neoadjuvant or adjuvant endocrine therapy, had radiologically confirmed disease progression (according to RECIST 1.1 criteria) during treatment or disease recurrence within 12 months after the end of treatment and has not received further endocrine therapy after the disease progression or disease recurrence.
   b) Recurrence more than 12 months after completion of neoadjuvant or adjuvant endocrine therapy, and subsequent recurrence again after receiving antiestrogens (such as tamoxifen, etc.) or aromatase inhibitors as the first-line endocrine therapy for advanced disease with radiologically confirmed progression (according to RECIST 1.1 criteria). No more than 1 line of endocrine therapy for advanced disease.
   c) For primary or metastatic disease, the subjects experienced recurrence after previously receiving antiestrogens (such as tamoxifen, etc.) or aromatase inhibitors as first-line endocrine therapy for metastatic disease with radiologically confirmed progression (according to RECIST 1.1 criteria). No more than 1 line of endocrine therapy for metastatic disease.
7) ECOG performance status scale: 0-1.
8) Expected survival time ≥ 3 months.
9) Female subjects should agree to take contraceptive measures (such as intrauterine devices [IUD], contraceptives or condoms) during the study and for 6 months after the study; serum or urine pregnancy test results should be negative within 7 days before enrollment, and the subjects must not be breastfeeding; male subjects should agree to take contraceptive measures during the study and for 6 months after the study.
10) Voluntary participation, written informed consent and good compliance.

### 2.3 Evaluation method and endpoints

Objective response rate (ORR), progression-free survival (PFS), disease control rate (DCR), duration of response (DOR), and overall survival (OS) of the combination treatment were evaluated according to RECIST 1.1 criteria.

### 2.4 Case 1

Female, 72 years old, pathological diagnosis with needle biopsy for right-breast lump suggesting invasive carcinoma. The patient received modified radical mastectomy for right-breast cancer lesion under general anesthesia and received postoperative examination. Regular report: (1) invasive ductal carcinoma of the right breast: grade III (score: 3 + 3 + 2 = 8), tumor size: about 2 × 1.1 × 0.8 cm, the tumor invaded extra-mammary adipose tissue, and intravascular cancer embolus was visible; (2) no cancer lesion was observed in the surgical margins of nipple, skin and basal margin; (3) 9/17 right axillary lymph nodes showed cancer metastasis. Immunohistochemistry report: ER (medium. weak, about 10%), PR (weak, about 5%), CerbB-2 (2+, suggesting Fish testing), Ki-67 (about 80%), P53+ E-Cadherin + EGFR-AR (strong, medium, about 80%), P63, CK5/6 and Calponin showed loss of myoepithelial cells. FISH testing: HER-2 negative. Clinical diagnosis: postoperative liver metastasis of right-breast cancer.

After the clinical diagnosis, the subject received 8 cycles of AC-T regimen chemotherapy (40 mg of doxorubicin liposome + 0.8 g of cyclophosphamide, 210 mg of paclitaxel liposome). Letrozole (2.5 mg, Qd) was administered orally for endocrine therapy within 7 months after chemotherapy. The chemotherapy was followed by postoperative radiotherapy with a PTV of 5000 cGy/25 times. CT results before enrollment suggested a high possibility of multiple metastases in the liver, considering disease progression.

After enrollment, tablets of the compound of formula **I** combined with fulvestrant injection were given, wherein the compound of formula **I** was administered at a dose of 20 mg (20 mg/tablet) daily. One week before the start of treatment, CT scan showed that the sum of the diameters of measurable target lesions (multiple metastases in the liver) was 51.6 mm. After drug administration, CT scanning was conducted regularly, and after 2 cycles of treatment, the sum of the diameters of target lesions was reduced to 43 mm. The outcome was evaluated as stable disease (SD), with a reduction of 16.67% and no new lesions.

### 2.5 Cases 2

Female, 34 years old, pathological diagnosis with needle biopsy for left-breast lump suggested malignant neoplasm, indicating invasive ductal carcinoma based on immunohistochemical results. Immunohistochemistry report: ER (about 80%, strong +), PR (about 70%, strong +), HER-2 (1+). Clinical diagnosis: malignant neoplasm in breast, secondary malignant neoplasm in lungs, secondary malignant neoplasm in liver, secondary malignant tumor in chest wall, secondary malignant neoplasm in axilla, secondary malignant neoplasm in bones.

After the clinical diagnosis, the subject received 3 cycles of EC regimen chemotherapy (150 mg/dose of epirubicin hydrochloride injection + 1 g/dose of cyclophosphamide injection). The EC regimen was followed by 8 cycles of TP regimen chemotherapy (210 mg/dose of paclitaxel injection + 30 mg/dose of cisplatin injection). The TP regimen was followed by capecitabine chemotherapy. The capecitabine chemotherapy was followed by endocrine therapy with tamoxifen in combination with goserelin. The endocrine therapy with tamoxifen in combination with goserelin was followed by endocrine therapy with letrozole in combination with goserelin. CT results before enrollment suggested the disease progression.

After enrollment, tablets of the compound of formula **I** combined with fulvestrant injection were given, wherein the compound of formula **I** was administered at a dose of 20 mg (20 mg/tablet) daily. CT scan before the start of treatment showed that the sum of the diameters of measurable target lesions (left breast node + bone metastasis) was 66.83 mm. After drug administration, CT scanning was conducted regularly, and after 4 cycles of treatment, the sum of the diameters of target lesions was reduced to 57.7 mm. The outcome was evaluated as stable disease (SD), with a reduction of 13.66% and no new lesions.

### Example 3 Evaluation of human breast cancer (BT-474) model

### 3.1 Methodology

BALB/c female nude mice (provided by Shanghai Sippr-BK Lab. Animal Co., Ltd.), SPF-grade, weight 18-25 g, aged about 8 weeks (tumor-bearing). Human breast cancer BT-474 cells (ATCC) were cultured in Hybri-Care medium containing 10% fetal bovine serum, 100 U/mL penicillin and 100 µg/mL through *in vitro* monolayer culture at 37 °C/5% CO₂. The cells were digested with trypsin-EDTA twice a week for passaging as per conventional practice. When cell confluence was 80%-90%, the cells were collected, counted, and inoculated. Estrogen tablets were implanted 3 days before cell inoculation, 0.2 mL of BT-474 cells (1 ×10⁷) were subcutaneously inoculated on the right back of each mouse, and grouping and administration was initiated when the mean tumor volume reached 127.19 mm³. The compound of formula **I** was dissolved in a vehicle: 0.01 mol/L HCl solution, 0.5% methylcellulose and deionized water were mixed well in a volume ratio of 20:30:100.

**Table 2 Animal grouping and administration regimen for BT-474 in vivo efficacy study**

| Drug | Dosage (mg/kg) | Number of animals (mice) | Volume of administration (µL/g) | Concentration (mg/mL) | Route of administration | Frequency of administration |
|---|---|---|---|---|---|---|
| Vehicle control group | - | 10 | 10 | - | Orally | Once daily for 28 days |
| Compound of formula I | 1 | 10 | 10 | 0.1 | Orally | Once daily for 28 days |
| Compound of formula I | 2 | 10 | 10 | 0.2 | Orally | Once daily for 28 days |
| Compound of formula I | 4 | 10 | 10 | 0.4 | Orally | Once daily for 28 days |

### 3.2 Tumor measurement and endpoints

The tumor volume was calculated using the following formula: V = 0.5a × b², where a and b represent the long diameter and short diameter of the tumor, respectively. The percentage value of T/C (%) reflects the tumor growth inhibition rate, and where T and C represent the tumor weight (tumor volume) of the treatment groups and the control group, respectively, on a certain day.

The tumor growth inhibition rate was calculated using the following formula: TGI (%) = [1 - (Tᵢ - T₀) / (Vᵢ - V₀)] × 100, where Tᵢ is the mean tumor volume of a certain treatment group on a certain day, T₀ is the mean tumor volume of the treatment group at the start of treatment, Vᵢ is the mean tumor volume of the vehicle control group on a certain day (same day as Tᵢ) and Vo is the mean tumor volume of the vehicle control group at the start of treatment. Tumor weights were measured at the end of treatment and percentage T_{weight}/C_{weight} was calculated, where T_{weight} and C_{weight} represent the tumor weights of the treatment and vehicle control groups, respectively.

### 3.3 Results

**Table 3 Evaluation of tumor inhibitory efficacy of the compound of formula I on BT-474 xenograft tumor model**

| Groups | Mean tumor volume (mm³) (day 0) | Mean tumor volume (mm³) (day 28) | T/C (%) | TGI (%) |
|---|---|---|---|---|
| Vehicle control group | 127 ± 11 | 397 ± 146 | ---- | ---- |
| Compound of formula I (1 mg/kg) | 127 ± 11 | 223 ± 50 | 56.09 | 64.56 |
| Compound of formula I (2 mg/kg) | 127 ± 11 | 159 ± 36 | 40.11 | 88.10 |
| Compound of formula I (4 mg/kg) | 127 ± 11 | 145 ± 40 | 36.52 | 93.35 |

The compound of the formula **I** showed significant anti-tumor efficacy in the BT-474 xenograft tumor model.

## Claims

1. Use of a compound of formula I or a pharmaceutically acceptable salt thereof in preparing a medicament for treating breast cancer in a patient,

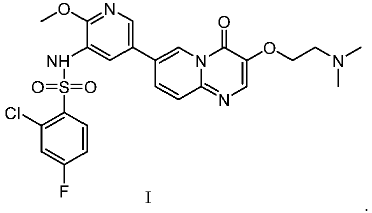

2. The use according to claim 1, wherein the breast cancer is a locally advanced recurrent and/or metastatic breast cancer.

3. The use according to claim 1 or 2, wherein the breast cancer is a hormone receptor-positive breast cancer; preferably, the hormone receptor-positive includes estrogen receptor-positive or progesterone receptor-positive.

4. The use according to any one of claims 1-3, wherein the breast cancer is a human epidermal growth factor receptor 2-negative breast cancer.

5. The use according to any one of claims 1-4, wherein the breast cancer is a breast cancer with a PIK3CA gene alteration; preferably, the PIK3CA gene alteration includes a PIK3CA gene mutation or a PIK3CA gene amplification.

6. The use according to any one of claims 1-5, wherein the patient with breast cancer is a hormone receptor-positive, human epidermal growth factor receptor 2-negative, PIK3CA gene altered, postmenopausal female patient with locally advanced recurrent and/or metastatic breast cancer.

7. The use according to any one of claims 1-6, wherein the patient with the breast cancer has previously received treatment with one or two or more prior therapeutic regimens.

8. The use according to any one of claims 1-7, wherein the compound of formula **I** or the pharmaceutically acceptable salt thereof is administered once daily to 3 times daily, or once every two days; preferably once daily.

9. The use according to any one of claims 1-8, wherein the compound of formula **I** or the pharmaceutically acceptable salt thereof is administered at a dose selected from the group consisting of 1-100 mg, preferably 5-50 mg, 10-50 mg, 10-40 mg, 10-15 mg and 20-30 mg; or the compound of formula **I** or the pharmaceutically acceptable salt thereof is administered at a daily dose selected from the group consisting of 1-100 mg, preferably 5-50 mg, 10-50 mg, 10-40 mg and 20-30 mg.

10. The use according to any one of claims 1-9, wherein the medicament for treating breast cancer in a patient further comprises fulvestrant or a pharmaceutically acceptable salt thereof.

11. A pharmaceutical combination comprising a compound of formula **I** or a pharmaceutically acceptable salt thereof, and fulvestrant or a pharmaceutically acceptable salt thereof,

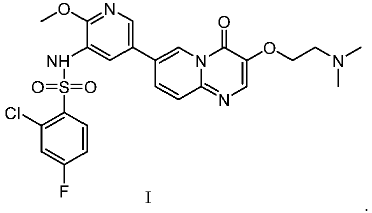

12. The pharmaceutical combination according to claim 11, wherein the pharmaceutical combination is for use in treating breast cancer in a patient; optionally, the pharmaceutical combination is administered simultaneously, separately or sequentially for treating breast cancer in a patient.

13. The pharmaceutical combination according to claim 11 or 12, wherein the compound of formula **I** or the pharmaceutically acceptable salt thereof, and fulvestrant or the pharmaceutically acceptable salt thereof are each in a form of a pharmaceutical composition, or optionally, can be administered simultaneously, sequentially, or at intervals.

14. The pharmaceutical combination according to any one of claims 11-13, wherein the pharmaceutical combination comprises a pharmaceutical composition of the compound of formula **I** or the pharmaceutically acceptable salt thereof at a single dose of 1-50 mg, and a pharmaceutical composition of fulvestrant or the pharmaceutically acceptable salt thereof at a single dose of 250-500 mg; preferably, the pharmaceutical composition of the compound of formula **I** or the pharmaceutically acceptable salt thereof has a single dose of 1 mg, 5 mg, or 20 mg.

15. The pharmaceutical combination according to any one of claims 11-14, wherein the pharmaceutical composition of the compound of formula **I** or the pharmaceutically acceptable salt thereof is a tablet; the pharmaceutical composition of fulvestrant or the pharmaceutically acceptable salt thereof is an injection.

16. Use of the pharmaceutical combination according to any one of claims 11-15 in treating breast cancer in a patient.

17. The use according to claim 16, wherein the breast cancer is a locally advanced recurrent and/or metastatic breast cancer.

18. The use according to claim 16 or 17, wherein the breast cancer is a hormone receptor-positive breast cancer; preferably, the hormone receptor-positive includes estrogen receptor-positive or progesterone receptor-positive.

19. The use according to any one of claims 16-18, wherein the breast cancer is a human epidermal growth factor receptor 2-negative breast cancer.

20. The use according to any one of claims 16-19, wherein the breast cancer is a breast cancer with a PIK3CA gene alteration; preferably, the PIK3CA gene alteration includes a PIK3CA gene mutation or a PIK3CA gene amplification.

21. The use according to any one of claims 16-20, wherein the patient with breast cancer is a hormone receptor-positive, human epidermal growth factor receptor 2-negative, PIK3CA gene altered, postmenopausal female patient with locally advanced recurrent and/or metastatic breast cancer.

22. The use according to any one of claims 16-21, wherein the patient with the breast cancer has previously received treatment with one or two or more prior therapeutic regimens.

23. The use according to any one of claims 16-22, wherein the compound of formula **I** or the pharmaceutically acceptable salt thereof and fulvestrant or the pharmaceutically acceptable salt thereof in the pharmaceutical combination are administered simultaneously, separately or sequentially.

24. The use according to any one of claims 16-23, wherein a treatment cycle of the breast cancer is 2-6 weeks, or preferably 4 weeks.

25. The use according to any one of claims 16-24, wherein the compound of formula **I** or the pharmaceutically acceptable salt thereof is administered once daily to 3 times daily, or once every two days, preferably once daily; wherein fulvestrant or the pharmaceutically acceptable salt thereof is administered once on days 1 and 15 in the first treatment cycle, and once on day 1 in each subsequent treatment cycle.

26. The use according to claim 24 or 25, wherein the compound of formula **I** or the pharmaceutically acceptable salt thereof is administered at a daily dose selected from the group consisting of 1-100 mg, preferably 5-50 mg, 10-50 mg, 10-40 mg and 20-30 mg; wherein fulvestrant or the pharmaceutically acceptable salt thereof is administered at a dose selected from the group consisting of 250-1000 mg, preferably 250-500 mg.

27. The use according to any one of claims 24-26, wherein within a single treatment cycle, the compound of formula **I** or the pharmaceutically acceptable salt thereof and fulvestrant or the pharmaceutically acceptable salt thereof have a mass ratio of (0.01-6):1, preferably (0.05-3):1, more preferably (0.1-3):1, even more preferably (0.2-2.5):1, still more preferably (0.3-2.0):1, and most preferably (0.5-1.8):1.

28. A kit comprising the pharmaceutical combination according to any one of claims 11-15 and an instruction for use of the compound of formula **I** or the pharmaceutically acceptable salt thereof in combination with fulvestrant or the pharmaceutically acceptable salt thereof in treating breast cancer.
